(19) **Europäisches Patentamt**

**European Patent Office**

**Office européen des brevets**

(11) Veröffentlichungsnummer: **0 431 478 B1**

(12) # EUROPÄISCHE PATENTSCHRIFT

(45) Veröffentlichungstag der Patentschrift: **07.09.94**

(21) Anmeldenummer: **90122905.4**

(22) Anmeldetag: **30.11.90**

(51) Int. Cl.⁵: **C07C 67/055**, C07C 69/15, B01J 37/00

(54) **Verfahren zur Herstellung von Vinylacetat.**

(30) Priorität: **05.12.89 DE 3940125**

(43) Veröffentlichungstag der Anmeldung:
**12.06.91 Patentblatt 91/24**

(45) Bekanntmachung des Hinweises auf die
Patenterteilung:
**07.09.94 Patentblatt 94/36**

(84) Benannte Vertragsstaaten:
**BE DE ES FR GB IT LU NL**

(56) Entgegenhaltungen:
**DE-A- 2 745 174**
**US-A- 3 939 199**

(73) Patentinhaber: **HOECHST AKTIENGESELL-SCHAFT**

**D-65926 Frankfurt (DE)**

(72) Erfinder: **Wunder, Friedrich, Dr.**
**Jahnstrasse 46**
**W-6093 Flörsheim am Main (DE)**
Erfinder: **Wirtz, Peter, Dr.**
**Wiesbadener Strasse 135**
**W-6240 Königstein/Taunus (DE)**
Erfinder: **Roscher, Günter, Dr.**
**Hölderlinstrasse 54**
**W-6233 Kelkheim/Taunus (DE)**
Erfinder: **Eichler, Klaus, Dr.**
**Im Traminer 10**
**W-6236 Eschborn (DE)**

## Beschreibung

Es ist bekannt, daß man Ethylen in der Gasphase mit Essigsäure und Sauerstoff oder sauerstoffhaltigen Gasen an Festbettkatalysatoren zu Vinylacetat umsetzen kann. Geeignete Katalysatoren enthalten einen Edelmetallanteil und einen Aktivatoranteil. Der Edelmetallanteil besteht vorzugsweise aus Palladium und/oder dessen Verbindungen; zusätzlich können noch Gold und/oder dessen Verbindungen anwesend sein (US-PS 3 939 199, DE-OS 2 100 778, US-PS 4 668 819). Der Aktivatoranteil besteht dabei aus Verbindungen von Elementen der 1. Hauptgruppe und/oder der 2. Hauptgruppe und/oder Cadmium. Bevorzugt ist Kalium als Element der 1. Hauptgruppe. Diese aktiven Komponenten werden in feiner Verteilung auf Träger aufgebracht, wobei als Trägermaterial im allgemeinen Kieselsäure oder Aluminiumoxid verwendet wird.

Die spezifische Oberfläche der Träger liegt im allgemeinen bei 40 - 350 $m^2/g$. Nach US-PS 3 939 199 soll das Gesamtporenvolumen bei 0,4 - 1,2 ml/g liegen, und davon sollen weniger als 10 % von "Mikroporen" mit einem Porendurchmesser unter 30 Å gebildet werden (Å = Angström = $10^{-8}$ cm). Geeignete Träger mit diesen Eigenschaften sind z.B. aerogenes $SiO_2$ oder aerogenes $SiO_2$-$Al_2O_3$-Gemisch. Die Trägerteilchen bei der Vinylacetatherstellung haben im allgemeinen die Form von Kugeln. Aber auch Tabletten und Zylinder wurden bereits eingesetzt.

In der noch nicht veröffentlichten deutschen Patentanmeldung P 39 19 524.4 wird ein Träger beschrieben, der aus $SiO_2$ oder $SiO_2$-$Al_2O_3$-Gemisch mit einer Oberfläche von 50 - 250 $m^2/g$ und einem Porenvolumen von 0,4 - 1,2 ml/g besteht und dessen Teilchen eine Korngröße von 4 bis 9 mm haben, wobei 5 bis 20 % des Porenvolumens des Trägers von Poren mit Radien von 200 bis 3000 Å und 50 bis 90 % des Porenvolumens von Poren mit Radien von 70 bis 100 Å gebildet wird.

Es wurde nun gefunden, daß es sehr vorteilhaft ist, wenn die Trägerteilchen mit Hilfe von Li-, Mg-, Al-, Zn- oder Mn-Carboxylaten als Bindemitteln gepreßt werden.

Gegenstand der Erfindung ist ein Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen sowie gegebenenfalls zusätzlich Gold und/oder Goldverbindungen sowie als Aktivatoren Alkaliverbindungen und gegebenenfalls zusätzlich Cadmiumverbindungen auf einem Träger enthält, der aus $SiO_2$ oder $SiO_2$-$Al_2O_3$-Gemisch mit einer Oberfläche von 50 - 250 $m^2/g$ und einem Porenvolumen von 0,4 - 1,2 ml/g besteht und dessen Teilchen eine Korngröße von 4 bis 9 mm haben, wobei 5 bis 20 % des Porenvolumens des Trägers von Poren mit Radien von 200 bis 3000 Å und 50 bis 90 % des Porenvolumens von Poren mit Radien von 70 bis 100 Å gebildet wird, dadurch gekennzeichnet, daß die Trägerteilchen mit Hilfe eines Li-, Mg-, Al-, Zn- oder Mn-Salzes einer $C_2$-$C_{20}$-Carboxylsäure oder eines Gemisches solcher Salze als Bindemittel gepreßt werden.

Das oder die Carboxylate werden in solchen Mengen eingesetzt, daß die Summe der Mengen von Li, Mg, Al, Zn, Mn, als Elemente gerechnet, 0,1 bis 5 Gew.-%, bezogen auf das Trägermaterial, vorzugsweise 0,3 bis 1,5 Gew.-% beträgt.

Vorzugsweise werden Al- oder Mg-Carboxylate, insbesondere Mg-Carboxylate eingesetzt. Die Carboxylsäuren haben vorzugsweise 12 bis 18 C-Atome. Die anspruchsgemäßen Trägerteilchen können beispielsweise wie folgt hergestellt werden: Zunächst werden glasige Mikrokugeln hergestellt, beispielsweise durch Flammenhydrolyse von Siliciumtetrachlorid oder einem Siliciumtetrachlorid-Aluminiumtrichlorid-Gemisch in einer Knallgasflamme (US-PS 3 939 199). Die Mikrokugeln können auch durch Schmelzen von feinstem $SiO_2$-Staub in einer ausreichend heißen Flamme und anschließende rasche Abkühlung hergestellt werden. Die auf eine der beiden Arten hergestellten Mikrokugeln haben eine Oberfläche von 100 - 300 $m^2/g$. Besonders geeignet sind Mikrokugeln mit einer Oberfläche von 150 - 250 $m^2/g$, die aus mindestens 95 Gew.-% $SiO_2$ und höchstens 5 Gew.-% $Al_2O_3$, insbesondere aus mindestens 99 Gew.-% $SiO_2$ und höchstens 1 Gew.-% $Al_2O_3$ bestehen. Mikrokugeln mit der genannten Oberfläche sind im Handel erhältlich, z.B. unter dem Namen ®Aerosil oder ®Cabosil oder als "hochdisperse Kieselsäure".

Aus den Mikrokugeln werden dann unter Zusatz von einem oder mehreren Carboxylaten von Li, Mg, Al, Zn oder Mn und unter Zusatz von organischen Füllstoffen (wie Zucker, Harnstoff, höhere Fettsäuren, längerkettige Paraffine, mikrokristalline Zellulose) und Gleitmitteln (wie Kaolin, Graphit, Metallseifen) Formkörper gepreßt, z.B. durch Tablettieren (nach einer Vorverdichtung) oder Extrudieren. Anschließend werden die Formkörper in $O_2$-haltigen Gasen geglüht. Bei Verwendung der Li-, Mg-, Al-, Zn- oder Mn-Salze höherer Carbonsäuren ($C_{16}$-$C_{20}$) wirken diese "Seifen" gleichzeitig bei der Tablettierung als Gleitmittel, so daß ein gesondertes Gleitmittel nicht zugesetzt werden muß. Die Oberfläche des fertigen Trägers, sein Porenvolumen und der Anteil des Porenvolumens, den Poren eines bestimmten Radius bilden ("Porenradienverteilung"), wird determiniert von der Art der Verformung (Tabletten, Strangpreßlinge usw.), der Temperatur und Dauer des Glühens, den relativen Mengen von Füllstoffen, Gleitmitteln und Mikrokugeln sowie der Oberfläche der Mikrokugeln. Welches die geeignetsten Werte für diese determinierenden Parameter sind, läßt sich durch einfache Vorversuche ermitteln.

Der nach dieser Methode erhaltene fertige Träger hat dann eine Oberfläche von 50 bis 250 $m^2/g$ und ein Porenvolumen von 0,4 bis 1,2 ml/g sowie eine Korngröße von 4 bis 9 mm (einstellbar durch Tablettieren oder Extrudieren von Formkörpern geeigneter Größe).

Durch Verwendung der anspruchsgemäßen Träger gelingt es, die Raum-Zeit-Ausbeute der Katalysatoren gegenüber konventionellen Trägern bei sonst gleichen Bedingungen (gleicher Gehalt an aktiven Substanzen auf dem Träger und gleichen Reaktionsbedingungen) wesentlich zu steigern und gleichzeitig die stärkste Nebenreaktion, die Verbrennung des Ethylens zu $CO_2$ um 75 % zu senken. Ebenso wird die als weitere Nebenreaktion ablaufende Ethylacetatbildung deutlich verringert. Durch diese Steigerung der Selektivität von ca. 92 % auf ca. 97 % können wesentliche Einsparungen erzielt und außerdem durch die Leistungssteigerung bei deutlich erhöhter Selektivität in Neuanlagen Katalysatormenge und Reaktorvolumen verringert werden, was zu beträchtlichen Verringerungen der Anlagekosten führt, oder bei bereits bestehenden Anlagen die Kapazität ohne Umbau wesentlich erhöht werden, so daß man die Investitionskosten für die Anlagenerweiterung einspart.

Bei der Oberfläche der genannten Träger handelt es sich stets um die sogenannte BET-Oberfläche, gemessen nach der Methode von Brunauer, Emmett und Teller. Sie gibt die Gesamtoberfläche von 1 g Trägermaterial an, d.h. die Summe aus der äußeren Oberfläche des Trägers und der inneren Oberfläche sämtlicher offener Poren. Das gesamte Porenvolumen und der Anteil davon, den Poren bestimmter Größe (z.B. diejenigen mit einem Durchmesser von 70 bis 100 Å) beitragen, läßt sich mit Hilfe der Quecksilber-Porosimetrie messen. Geeignete Meßapparaturen werden z.B. von den Firmen Carlo Erba oder Micromeritics hergestellt.

Die katalytisch aktiven Substanzen werden in üblicher Weise auf den Träger aufgebracht, beispielsweise durch Tränken des Trägers mit einer Lösung der aktiven Substanzen, anschließende Trocknung und gegebenenfalls Reduktion. Jedoch kann man die aktiven Substanzen auch beispielsweise durch Ausfällung auf dem Träger, durch Aufsprühen, Aufdampfen oder Tauchen aufbringen.

Als Lösungsmittel für die katalytisch aktiven Substanzen sind vor allem unsubstituierte Carbonsäuren mit 2 bis 10 Kohlenstoffatomen im Molekül, wie Essigsäure, Propionsäure, n- und iso-Buttersäure und die verschiedenen Valeriansäuren geeignet. Wegen ihrer physikalischen Eigenschaften und auch aus wirtschaftlichen Gründen wird vorzugsweise Essigsäure als Lösungsmittel eingesetzt. Die zusätzliche Verwendung eines inerten Lösungsmittels ist dann zweckmäßig, wenn die Substanzen in der Carbonsäure nicht ausreichend löslich sind. So läßt sich z.B. Palladiumchlorid in einer wäßrigen Essigsäure wesentlich besser lösen als in Eisessig. Als zusätzliche Lösungsmittel kommen diejenigen in Betracht, die inert und mit der Carbonsäure mischbar sind. Genannt seien neben Wasser beispielsweise Ketone wie Aceton und Acetylaceton, ferner Ether wie Tetrahydrofuran oder Dioxan, aber auch Kohlenwasserstoffe wie Benzol.

Der Katalysator enthält als Edelmetallkomponente Palladium und/oder dessen Verbindungen und als Aktivatorkomponente Alkaliverbindungen. Als zusätzliche Edelmetallkomponente kann er Gold und/oder dessen Verbindungen enthalten, und als zusätzliche Aktivatorkomponente kann er Cadmiumverbindungen enthalten.

Als Verbindungen des Palladiums kommen alle Salze und Komplexe in Betracht, die löslich (sowie gegebenenfalls reduzierbar) sind und im fertigen Katalysator keine desaktivierenden Stoffe wie Halogen oder Schwefel hinterlassen. Besonders geeignet sind die Carboxylate, vorzugsweise die Salze der aliphatischen Monocarbonsäuren mit 1 bis 5 Kohlenstoffatomen, etwa das Acetat, das Propionat oder das Butyrat. Weiter sind beispielsweise geeignet das Nitrat, Nitrit, Oxidhydrat, Oxalat, Acetylacetonat oder das Acetoacetat. Aber auch Verbindungen wie das Sulfat und die Halogenide können verwendet werden, wenn man dafür Sorge trägt, daß der Sulfatrest, z.B. durch Fällen mit Bariumacetat, oder das Halogen, z.B. durch Fällen mit Silbernitrat, vor der Tränkung entfernt wird, so daß das Sulfat- oder Halogenanion nicht auf den Träger gerät. Wegen seiner Löslichkeit und seiner Zugänglichkeit ist Palladiumacetat die besonders bevorzugte Palladiumverbindung.

Im allgemeinen liegt der Gehalt an Palladium im Katalysator bei 1,0 bis 3 Gew.-%, vorzugsweise bei 1,5 bis 2,5 Gew.-%, insbesondere bei 2 bis 2,5 Gew.-%, bezogen auf die Gesamtmasse des Trägerkatalysators.

Neben Palladium und/oder dessen Verbindungen können noch zusätzlich Gold und/oder dessen Verbindungen anwesend sein. Eine besonders geeignete Goldverbindung ist Bariumacetoaurat. Im allgemeinen wird Gold bzw. eine seiner Verbindungen, falls es eingesetzt wird, in einem Anteil von 0,2 bis 0,7 Gew.-% zugegeben, bezogen auf die Gesamtmasse des Trägerkatalysators, wobei im Falle einer Goldverbindung nur der Goldanteil gerechnet wird.

Als Aktivatoren enthält der Katalysator Alkaliverbindungen und gegebenenfalls zusätzlich Cadmiumverbindungen. Geeignet sind beispielsweise Alkalicarboxylate wie etwa Kaliumacetat, Natriumacetat, Lithiumacetat, Natriumpropionat. Geeignet sind auch solche Alkaliverbindungen, die unter Reaktionsbedingungen in die Carboxylate übergehen, wie etwa Hydroxide, Oxide, Carbonate. Als Verbindungen des Cadmiums kommen solche in Frage, die kein Halogen oder Schwefel enthalten, beispielsweise Carboxylat (bevorzugt), Oxid, Hydroxid, Carbonat, Citrat, Tartrat, Nitrat, Acetylacetonat, Benzoylacetonat, Acetoacetat. Besonders geeignet ist Cadmiumacetat. Auch Gemische verschiedener Aktivatoren lassen sich einsetzen. Jeder einzelne Aktivator wird im allgemeinen in einem Anteil von 0,5 - 4 Gew.-% zugegeben, wobei nur der Metallanteil des Aktivators gerechnet wird, und zwar auf die Gesamtmasse des Trägerkatalysators bezogen.

Bevorzugt sind folgende Katalysatoren:

Palladium/Cadmium/Alkalielement sowie Palladium/Gold/Alkalielement, wobei Palladium bzw. Gold als Metalle oder Verbindungen im fertigen Katalysator vorliegen können und wobei als Alkalielement Kalium bevorzugt ist (in Form eines Carboxy- ylats). Das Verhältnis K:Pd bzw. K:(Pd + Au) ist dabei vorzugsweise 0,7:1 bis 2:1. Das Verhältnis Cd:Pd bzw. Cd:(Pd + Au) ist vorzugsweise 0,6:1 bis 2:1, insbesondere 0,6:1 bis 0,9:1. Dabei werden Pd, Au, Cd, K stets als Elemente gerechnet, d.h. z.B. nur die Metallanteile von Pd-Acetat, Cd-Acetat und K-Acetat auf dem Träger miteinander verglichen.

Besonders bevorzugt sind die Katalysatoren Palladiumacetat/Cadmiumacetat/Kaliumacetat sowie Palladiumacetat/Bariumacetoaurat/Kaliumacetat.

Die Tränkung des Katalysatorträgers mit der Lösung der aktiven Komponenten wird vorzugsweise so vorgenommen, daß das Trägermaterial mit der Lösung überschichtet und die überschüssige Lösung dann abgegossen oder abfiltriert wird. Mit Rücksicht auf Lösungsverluste ist es vorteilhaft, nur die dem integralen Porenvolumen des Katalysatorträgers entsprechende Lösung einzusetzen und sorgfältig durchzumischen, damit die Teilchen des Trägermaterials gleichmäßig benetzt werden. Diese Durchmischung läßt sich z.B. durch Rühren erreichen. Es ist zweckmäßig, den Tränkungsvorgang und das Durchmischen gleichzeitig durchzuführen, beispielsweise in einer Drehtrommel oder einem Taumeltrockner, wobei sich die Trocknung sofort anschließen kann. Weiterhin ist es zweckmäßig, die Menge und die Zusammensetzung der zum Tränken des Katalysatorträgers verwendeten Lösung so zu bemessen, daß sie dem Porenvolumen des Trägermaterials entspricht und daß durch einmaliges Tränken die gewünschte Menge aktiver Stoffe aufgebracht wird.

Die Trocknung des mit der Lösung der aktiven Stoffe getränkten Katalysatorträgers wird vorzugsweise unter vermindertem Druck durchgeführt. Die Temperatur bei der Trocknung sollte unter 120°C, vorzugsweise unter 90°C liegen. Weiterhin empfiehlt es sich im allgemeinen, die Trocknung in einem Inertgasstrom, beispielsweise in einem Stickstoff- oder Kohlendioxidstrom vorzunehmen. Der Lösungsmittel-Restgehalt nach der Trocknung sollte vorzugsweise weniger als 8 Gew.-%, insbesondere weniger als 6 Gew.-% betragen.

Falls eine Reduktion der Palladiumverbindungen (und ggf. der Goldverbindungen) durchgeführt wird, was manchmal nützlich sein kann, so kann diese im Vakuum, bei Normaldruck oder bei erhöhtem Druck bis zu 10 bar, ausgeführt werden. Dabei empfiehlt es sich, das Reduktionsmittel um so stärker mit einem Inertgas zu verdünnen, je höher der Druck ist. Die Reduktionstemperatur liegt zwischen 40 und 260°C, vorzugsweise zwischen 70 und 200°C. Im allgemeinen ist es zweckmäßig, für die Reduktion ein Inertgas-Reduktionsmittel-Gemisch zu verwenden, das 0,01 bis 50 Vol.-%, vorzugsweise 0,5 bis 20 Vol.-% Reduktionsmittel enthält. Als Inertgas können beispielsweise Stickstoff, Kohlendioxid oder ein Edelgas verwendet werden. Als Reduktionsmittel kommen beispielsweise Wasserstoff, Methanol, Formaldehyd, Ethylen, Propylen, Isobutylen, Butylen und andere Olefine in Frage. Die Menge des Reduktionsmittels richtet sich nach der Menge des Palladiums und ggf. des eingesetzten Goldes; das Reduktionsäquivalent soll mindestens das 1- bis 1,5-fache des Oxidationsäquiva- lents betragen, jedoch schaden größere Mengen Reduktionsmittel nicht. Beispielsweise soll auf 1 Mol Palladium mindestens 1 Mol Wasserstoff verwendet werden. Die Reduktion kann im Anschluß an die Trocknung in der gleichen Anlage vorgenommen werden.

Die Herstellung des Vinylacetats erfolgt im allgemeinen durch Leiten von Essigsäure, Ethylen und Sauerstoff oder Sauerstoff enthaltenden Gasen bei Temperaturen von 100 bis 220°C, vorzugsweise 120 bis 200°C, und bei Drucken von 1 bis 25 bar, vorzugsweise 1 bis 20 bar, über den fertigen Katalysator, wobei nicht-umgesetzte Komponenten im Kreis geführt werden können. Zweckmäßig hält man die Sauerstoffkonzentration unter 10 Vol.-% (bezogen auf das essigsäurefreie Gasgemisch). Unter Umständen ist jedoch auch eine Verdünnung mit inerten Gasen wie Stickstoff oder Kohlendioxid vorteilhaft. Besonders $CO_2$ eignet sich zur Verdün- nung bei Kreisprozessen, da es in geringen Mengen während der Reaktion gebildet wird.

Die folgenden Beispiele sollen die Erfindung erläutern.

**Vergleichsbeispiel 1**
(Kugelförmige Trägerteilchen aus konventionellem Kieselgel)

Es wurden 200 g eines bindemittelfreien Kieselsäureträgers eingesetzt, der aus getemperten (800°C) Kieselgel-Kugeln von 5 - 8 mm Durchmesser bestand. Der (handelsübliche) Träger aus diesen kugelförmigen Teilchen hatte eine BET-Oberfläche von 169 $m^2$/g und ein Porenvolumen von 0,48 ml/g, das zu 8% von Poren mit 70 - 100 Å Radius und zu 29 % von Poren mit 200 - 3000 Å Radius gebildet wurde. Der Träger wurde mit einer (diesem Porenvolumen entsprechenden) Lösung von 11,5 g Pd-Acetat, 10,0 g Cd-Acetat und 10,8 g K-Acetat in 66 ml Eisessig getränkt und bei 60°C unter Stickstoff bei einem Druck von 200 mbar bis zu einem Lösungsmittel-Restgehalt von 2 Gew.-% getrocknet. Dies ergab eine Dotierung von 2,3 Gew.-% Pd, 1,8 Gew.-% Cd, 2,0 Gew.-% K (Cd:Pd = 0,78:1, K:Pd = 0,87:1).

Es wurden 50 ml des fertigen Katalysators in ein Reaktionsrohr von 8 mm Innendurchmesser und einer Länge von 1,5 m eingefüllt. Dann wurde bei einem Druck von 8 bar (Reaktoreingang) und einer Katalysatortemperatur von 150°C das umzusetzende Gas über den Katalysator geleitet. Dieses Gas bestand am Reaktoreingang aus 27 Vol.-% Ethylen, 55 Vol.-% $N_2$, 12 Vol.-% Essigsäure und 6 Vol.-% $O_2$. Die Ergebnisse sind aus der Tabelle ersichtlich.

**Vergleichsbeispiel 2**
(Kugelförmige Trägerteilchen aus konventionellem $SiO_2$)

Es wurden 200 g eines Kieselsäureträgers eingesetzt, der aus gebranntem und dann mit HCl gewaschenem Bentonit ($SiO_2$-Gehalt nach dieser Wäsche 96 Gew.-%) zu Kugeln von 5 - 6 mm Durchmesser ohne Bindemittel gepreßt worden war. Der Träger aus diesen kugelförmigen Teilchen hatte eine BET-Oberfläche von 121 $m^2$/g und ein Porenvolumen von 0,66 ml/g, das zu 21 % von Poren mit 70 - 100 Å Radius und zu 42 % von Poren mit 200 - 3000 Å Radius gebildet wurde. Die Trägerteilchen wurden wie in Vergleichsbeispiel 1 getränkt (nur daß 114 ml statt 65 ml Eisessig verwendet wurden) und getrocknet, so daß die gleiche Dotierung wie dort vorlag. Anschließend wurde der Katalysator wie in Vergleichsbeispiel 1 getestet. Die Ergebnisse sind aus der Tabelle ersichtlich.

**Vergleichsbeispiel 3**

Es wurde zunächst aus $SiO_2$-Mikrokugeln mit einer Oberfläche von 200 $m^2$/g sowie mikrokristalliner Cellulose als Füllstoff, Graphit als Gleitmittel und Kaolin als Bindemittel ein Träger hergestellt. Der fertige Träger hatte ein Porenvolumen von 0,80 ml/g, das zu 62 % von Poren mit 70 - 100 Å Radius und zu 9 % von Poren mit 200 - 3000 Å Radius gebildet wurde. Die Trägerteilchen hatten die Form von Zylindern mit gewölbten Stirnflächen (6 mm Durchmesser und 6 mm Höhe; die Form ist ähnlich der Form der bekannten Arzneimittelkapseln). Die Oberfläche der Trägerteilchen betrug 185 $m^2$/g.

Die Trägerteilchen (200 g) wurden wie in Vergleichsbeispiel 1 getränkt (nur daß 141 ml statt 66 ml Eisessig verwendet wurden) und getrocknet, so daß die gleiche Dotierung wie dort vorlag. Anschließend wurde der Katalysator wie in Vergleichsbeispiel 1 getestet. Die Ergebnisse sind aus der Tabelle ersichtlich.

**Vergleichsbeispiel 4**

Es wurde zunächst aus $SiO_2$-$Al_2O_3$-Mikrokugeln (97 Gew.-% $SiO_2$, 3 Gew.-% $Al_2O_3$) mit einer Oberfläche von 170 $m^2$/g und Zucker als Füllstoff, Graphit als Gleitmittel sowie Kaolin als Bindemittel ein Träger hergestellt. Der fertige Träger hatte ein Porenvolumen von 0,75 ml/g, das zu 58 % von Poren mit 70 - 100 Å Radius und zu 12 % von Poren mit 200 - 3000 Å Radius gebildet wurde. Die Trägerteilchen hatten dieselbe Form und Größe wie in Vergleichsbeispiel 3, jedoch hatten sie jetzt eine Oberfläche von 132 $m^2$/g. Die Trägerteilchen (200 g) wurden wie in Vergleichsbeispiel 1 getränkt (nur daß 131 ml statt 66 ml Eisessig verwendet wurden) und getrocknet, so daß die gleiche Dotierung wie dort vorlag. Anschließend wurde der Katalysator wie in Vergleichsbeispiel 1 getestet. Die Ergebnisse sind aus der Tabelle ersichtlich.

**Beispiel 1**

Der Träger wurde wie im Vergleichsbeispiel 3 hergestellt, nur daß ca. 10 Gew.-% Mg-stearat als Bindemittel verwendet wurde; der fertige Träger enthielt 0,4 Gew.-% Mg. Er hatte eine Oberfläche von 186 $m^2$/g und ein Porenvolumen von 0,8 ml/g, wobei 78 % des Porenvolumens von Poren mit Radien von 70 - 100 Å und 16 % des Porenvolumens von Poren mit Radien von 200 - 3000 Å gebildet wurden. Die Trägerteilchen hatten die gleiche Form und Größe wie in Vergleichsbeispiel 3 und 4.

Die Trägerteilchen (200 g) wurden wie in Vergleichsbeispiel 1 getränkt (nur daß wegen des höheren Porenvolumens 141 ml statt 66 ml Eisessig verwendet wurden) und getrocknet, so daß die gleiche Dotierung wie dort vorlag. Anschließend wurde der Katalysator wie in Vergleichsbeispiel 1 getestet. Die Ergebnisse sind aus der Tabelle ersichtlich.

**Beispiel 2**

Der Träger wurde wie in Beispiel 1 hergestellt, nur daß an Stelle von Mg-stearat jetzt 10 Gew.-% Al-stearat eingesetzt wurden; der fertige Träger enthielt 0,3 Gew.-% Al. Er hatte eine Oberfläche von 164 m²/g und ein Porenvolumen von 0,91 ml/g, wobei 76 % des Porenvolumens von Poren mit Radien von 70 - 100 Å und 18 % des Porenvolumens von Poren mit Radien von 200 - 3000 Å gebildet wurden. Die Trägerteilchen hatten die gleiche Form und Größe wie in Beispiel 1 sowie den Vergleichsbeispielen 3 und 4.

Die Trägerteilchen (200 g) wurden wie in Vergleichsbeispiel 1 getränkt (nur daß wegen des höheren Porenvolumens 160 ml statt 66 ml Eisessig verwendet wurden) und getrocknet, so daß die gleiche Dotierung wie dort vorlag. Anschließend wurde der Katalysator wie in Vergleichsbeispiel 1 getestet. Die Ergebnisse sind aus der Tabelle ersichtlich.

**Tabelle**

|  | Vergleichs-beispiel 1 | Vergleichs-beispiel 2 | Vergleichs-beispiel 3 | Vergleichs-beispiel 4 | Beispiel 1 | Beispiel 2 |
|---|---|---|---|---|---|---|
| RZA (g/l·h) | 440 | 742 | 773 | 763 | 838 | 790 |
| Verbrennung (%) | 14 | 15 | 6 | 8 | 5,4 | 4,6 |
| Ethylacetatgehalt (ppm) | 260 | 280 | 160 | 184 | 190 | 218 |

"RZA" bedeutet die Raum-Zeit-Ausbeute; "Verbrennung (%)" bedeutet den Prozentsatz des umgesetzten Ethylens, der in $CO_2$ umgewandelt wird, "Ethylacetatgehalt" bezieht sich auf den Gehalt des kondensierten Teils des Reaktionsproduktes an Ethylacetat.

**Patentansprüche**

1. Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen sowie gegebenenfalls zusätzlich Gold und/oder Goldverbindungen sowie als Aktivatoren Alkaliverbindungen und gegebenenfalls zusätzlich Cadmiumverbindungen auf einem Träger enthält, der aus $SiO_2$ oder $SiO_2$-$Al_2O_3$-Gemisch mit einer Oberfläche von 50 - 250 $m^2$/g und einem Porenvolumen von 0,4 - 1,2 ml/g besteht und dessen Teilchen eine Korngröße von 4 bis 9 mm haben, wobei 5 bis 20 % des Porenvolumens des Trägers von Poren mit Radien von 200 bis 3000 Å und 50 bis 90 % des Porenvolumens von Poren mit Radien von 70 bis 100 Å gebildet wird, dadurch gekennzeichnet, daß die Trägerteilchen mit Hilfe eines Li-, Mg-, Al-, Zn- oder Mn-Salzes einer $C_2$-$C_{20}$-Carboxylsäure oder eines Gemisches solcher Salze als Bindemittel gepreßt werden.

2. Verfahren nach Anspruch 1, dadurch gekennzeichnet, daß Al- oder Mg-Carboxylate als Bindemittel eingesetzt werden.

3. Verfahren nach Anspruch 1, dadurch gekennzeichnet, daß Mg-Carboxylate als Bindemittel eingesetzt werden.

**Claims**

1. A process for the preparation of vinyl acetate in the gas phase from ethylene, acetic acid and oxygen or oxygen-containing gases on a catalyst which contains palladium and/or its compounds and, if desired, additionally gold and/or gold compounds and which contains as activators alkali metal compounds and, if desired, additionally cadmium compounds on a support which is composed of $SiO_2$ or an $SiO_2$-$Al_2O_3$ mixture having a surface area of 50 - 250 $m^2$/g and a pore volume of 0.4 - 1.2 ml/g and whose particles have a particle size of 4 to 9 mm, 5 to 20% of the pore volume of the support being formed of pores having radii of 200 to 3000 Å and 50 to 90% of the pore volume being formed of pores having radii of 70 to 100 Å, which comprises compressing the support particles with the aid of an Li, Mg, Al, Zn or Mn salt of a $C_2$-$C_{20}$ carboxylic acid or a mixture of such salts as binder.

2. The process as claimed in claim 1, wherein carboxylates of Al or Mg are employed as binders.

3. The process as claimed in claim 1, wherein Mg carboxylates are employed as binders.

**Revendications**

1. Procédé de préparation d'acétate de vinyle en phase gazeuse à partir d'éthylène, d'acide acétique et d'oxygène ou de gaz contenant de l'oxygène, sur un catalyseur comprenant sur un support du palladium et/ou des composés de palladium et le cas échéant en outre de l'or et/ou des composés d'or, ainsi que des composés alcalins comme activants et le cas échéant en outre des composés de cadmium, support qui est formé de $SiO_2$ ou d'un mélange $SiO_2$-$Al_2O_3$ de surface spécifique 50 - 250 $m^2$/g avec un volume de pores de 0,4 - 1,2 ml/g, en particules de 4 à 9 mm, et dont 5 à 20% du volume des pores sont formés de pores ayant des rayons de 200 à 3000 Å et 50 à 90% de pores ayant des rayons de 70 à 100 Å, procédé caractérisé en ce que les particules du support sont pressées, à l'aide, comme liant, d'un sel de Li, Mg, Al, Zn ou Mn d'un acide carboxylique en $C_2$-$C_{20}$ ou d'un mélange de tels sels.

2. Procédé selon la revendication 1, caractérisé en ce que l'on utilise comme liant un carboxylate d'aluminium ou de magnésium.

3. Procédé selon la revendication 1, caractérisé en ce que l'on utilise comme liant un carboxylate de magnésium.